# EUROPÄISCHE PATENTSCHRIFT

(11) **EP 0 966 289 B1**
(45) Veröffentlichungstag und Bekanntmachung des Hinweises auf die Patenterteilung: **09.04.2003**
(21) Anmeldenummer: 98947504.1
(22) Anmeldetag: 02.09.1998
(51) Int. Cl.: A61K 31/58, A61P 1/16

(54) **BUDESONID ZUR BEHANDLUNG VON CHOLESTATISCHEN LEBERERKRANKUNGEN**
BUDESONIDE FOR THE TREATMENT OF CHOLESTATIC LIVER DISEASES
BUDESONIDE POUR LE TRAITEMENT D'AFFECTIONS HEPATIQUES CHOLESTATIQUES

(30) Priorität: 26.09.1997 DE 19742612
(43) Veröffentlichungstag der Anmeldung: 29.12.1999
(73) Patentinhaber: DR. FALK PHARMA GMBH, D-79108 Freiburg (DE)
(72) Erfinder: LEUSCHNER, Maria, D-60320 Frankfurt (DE)
(74) Vertreter: Best, Michael, Dr.
(86) Internationale Anmeldenummer: EP9805568
(87) Internationale Veröffentlichungsnummer: WO99016450

(56) Entgegenhaltungen:
- C.VETTER: "Wichtige Neuerungen bei Hepatitiden und cholestatischen Lebererkrankungen" AKTUELLE WISSENSCHAFT FÜR KLINIK UND PRAXIS, SATELLITENSYMPOSIUM, "AKTUELLE HEPATOLOGIE - DIAGNOSTISCHE UND THERAPEUTISCHE FORTSCHRITTE 1997", 5. April 1997, Seiten 2-3, XP002093555 Wiesbaden in der Anmeldung erwähnt
- U.LEUSCHNER ET AL.: ""Ursodeoxycholic acid in combination with prednisolone or budenoside in the therapy of primary biliary cirrhosis" in: BILE ACIDS IN HEPATOBILIARY DISEASES: BASIC RESEARCH AND CLINICAL APPLICATIONS (Proceedings of the Falk Symposium No. 93 (XIV International Bile Acid Meeting), Freiburg, 22-24/10/96" 1997 , KLUWER ACADEMIC PUBLISHERS , DORDRECHT XP002093590 in der Anmeldung erwähnt siehe Seite 299 - Seite 302
- E.G.TAMBUSCH ET AL.: "Immunsuppressive Therapie der autoimmunen Lebererkrankungen" INTERNIST, Bd. 38, Nr. 6, Juni 1997, Seiten 574-581, XP002093556
- N.LARUSSO: "Search for medical treatment for primary biliary cirrhosis" LANCET, Bd. 350, Nr. 9084, 11. Oktober 1997, Seite 1046 XP002093557
- A.SCHÜLER ET AL.: "TREATMENT OF AUTOIMMUNE HEPATITIS WITH BUDESONIDE" HEPATOLOGY, Bd. 22, Nr. 4 Part 2, Oktober 1995, Seite 488A XP002093558
- F.P.VLEGGAAR ET AL.: "TREATMENT WITH BUDESONIDE OR PREDNISONE IN COMBINATION WITH URSODEOXYCHOLIC ACID (UDCA) IN PRIMARY SCLEROSING CHOLANGITIS (PSC): A RANDOMIZED CONTROLLED PILOT STUDY" HEPATOLOGY, Bd. 28, Nr. 4 Part 2, Oktober 1998, Seite 647A XP002093559
- A. DANIELSSON ET AL.: "Oral budesonide for treatment of autoimmune chronic active hepatitis" ALIMENT PHARMACOL THER, Bd. 8, Nr. 6, Dezember 1994, Seiten 585-590, XP002093560
- A.J.CZAJA: "DIAGNOSIS AND THERAPY OF AUTOIMMUNE LIVER DISEASE" THE MEDICAL CLINICS OF NORTH AMERICA, Bd. 80, Nr. 5, September 1996, Seiten 973-994, XP002093561

## Beschreibung

Die vorliegende Erfindung betrifft die Verwendung des bekannten Arzneistoffs Budesonid zur Behandlung bestimmter Lebererkrankungen.

Im Fall von nicht cholestatischen Lebererkrankungen ist die Therapie mit Immunsuppressiva, darunter Corticosteroiden wie Prednisolon oder Budesonid etabliert (Danielsson et al., Aliment. Pharmacol. Ther., 1994, 8, 585-590). Von den nicht cholestatischen Erkrankungen unterschieden werden jedoch cholestatische Lebererkrankungen wie primäre biliäre Cirrhose (PBC), primäre sclerosierende Cholangitis (PSC) und autoimmune Cholangitis (AC), bei denen die Therapie mit solchen Immunsuppressiva im allgemeinen als nicht erfolgversprechend angesehen wird.

So wurden für die vorstehend genannten Erkrankungen bereits Therapieversuche mit Prednisolon durchgeführt (Mitchison et al., Hepatology, 1989, 4, 420-429; Mitchison et al., Hepatology, 1992, 15, 336-344), die Therapie mit Prednisolon wird jedoch kontrovers diskutiert. Es wird über eine nicht eindeutige Wirksamkeit bei gleichzeitig schweren steroidassoziierten Nebenwirkungen berichtet. Aus diesem Grund erschien eine Therapie mit Glucocorticoiden bei PBC, PSC und AC bislang nicht sinnvoll (Paumgartner & Beuers, In: Acute and chronic liver diseases, 1996, 96-106; Hepatologie (Hrsg. Gerok & Blum), 1995, Seite 435 und 439; Praktische Gastroenterologie, (Hrsg. Layer et al.), 1996, 397-398).

Budesonid ist ein Steroid das im Darm zu 52% bis 79% innerhalb von vier bis sechs Stunden resorbiert wird. Seine Affinität zu den Corticoidrezeptoren ist 15 mal größer als von Prednisolon, Der First-Pass Metabolismus durch die Leber beträgt 80% bis 90%. Die systemische Bioverfügbarkeit liegt bei etwa 10%. Aufgrund dieser Eigenschaften gehört Budesonid zwar zu den Steroiden mit äußerst geringen Nebenwirkungen, der hohe First-Pass Metabolismus und die geringe systemische Bioverfügbarkeit von Budesonid scheinen eine Behandlung von cholestatischen Lebererkrankungen aber auszuschließen.

Es entspricht daher auch der allgemeinen Auffassung, daß die einzige Möglichkeit einer wirkungsvollen medikamentösen Therapie insbesondere der PBC und der PSC die Verabreichung von Ursodesoxycholsäure darstellt, die allerdings die Erkrankung nicht zum Ausheilen bringt (Aktuelle Wissenschaft für Klinik und Praxis, Satellitensymposium, "Aktuelle Hepatologie - Diagnostische und therapeutische Fortschritte 1997", Wiesbaden, 5. April 1997, Seite 3). Zur Unterstützung von Ursodesoxycholsäure wurden verschiedene Verbindungen vorgeschlagen, unter anderem auch Budesonid (Aktuelle Wissenschaft für Klinik und Praxis, Satellitensymposium, "Aktuelle Hepatologie - Diagnostische und therapeutische Fortschritte 1997", Wiesbaden, 5. April 1997, Seite 3; U. Leuschner et al., Ursodeoxycholic acid in combination with prednisolone or budesonide in the therapy of primary biliary cirrhosis. In: Bile acids in Hepatobiliary Diseases: Basic Research and Clinical Application. S. 299-302. Kluwer Academic Publishers, Dordrecht, 1997. Die Verwendung von Budesonid alleine und nicht als Unterstützung von Ursodesoxycholsäure wurde als nicht erfolgversprechend angesehen und deshalb auch im Stand der Technik noch nicht vorgeschlagen.

Trotz der guten Behandlungserfolge bei der Behandlung von cholestatischen Lebererkrankungen mit Ursodesoxycholsäure besteht ein Bedarf nach weiteren Arzneimitteln, die ähnlich gute Ergebnisse bei der Behandlung cholestatischer Lebererkrankungen erzielen. Aufgabe der Erfindung ist es daher, ein Arzneimittel zur Verfügung zu stellen, das cholestatische Lebererkrankungen insbesondere primäre biliäre Cirrhose, primäre sclerosierende Cholangitis und autoimmune Cholangitis erfolgreich behandeln kann.

Die Lösung dieser Aufgabe beruht auf dem überraschenden Befund, daß das Glucocorticoid Budesonid, von dem bisher angenommen wurde, daß es allenfalls unterstützend bei der Behandlung von cholestatischen Erkrankungen mit Ursodesoxycholsäure wirkt, auch ohne gleichzeitige Verabreichung der Ursodesoxycholsäure ausgezeichnete Wirksamkeit gegen cholestatische Lebererkrankungen zeigt. Erfindungsgemäß wird daher die Verwendung von Budesonid zur Behandlung cholestatischer Lebererkrankungen insbesondere zur Behandlung der primären biliären Cirrhose, der primären sclerosierenden Cholangitis und der autoimmunen Cholangitis zur Verfügung gestellt.

Erfindungsgemäß wird Budesonid nicht zur Unterstützung der Behandlung einer cholestatischen Lebererkrankung mit Ursodesoxycholsäure verabreicht, sondern stellt den eigentlichen Wirkstoff zur Behandlung der Erkrankungen dar. Arzneimittel oder Therapiepläne, die die gleichzeitige oder zeitlich versetzte Verabreichung von Budesonid und Ursodesoxycholsäure so vorsehen, daß Budesonid und Ursodesoxycholsäure gemeinsam wirken, sind daher nicht Gegenstand der Erfindung.

Das Budesonid kann auf an sich bekannte Art und Weise zu Arzneimitteln für Säuger, bevorzugt Menschen, formuliert werden. In den Arzneimitteln liegt Budesonid im Gemisch mit einem pharmazeutischen organischen oder anorganischen Träger, der für die enterale oder parenterale Verabreichung geeignet ist, vor. Die orale Verabreichung der erfindungsgemäßen Arzneimittel über Tabletten, Kapseln, Pulver oder in flüssiger Form wie als Suspension, in Lösung, als Emulsion oder als Sirup ist besonders bevorzugt.

Bei der Formulierung als Tabletten werden übliche Arzneimittelträger wie Lactose, mikrokristalline Cellulose, Stärke und wasserfreie Kieselsäure, Schmiermittel wie hydriertes Castoröl, Magnesiumstearat, Natriumlaurylsulfat und Talk, sowie Bindemittel wie Stärke, Glucose, Gummiarabicum und Mannit verwendet.

Wenn die Zusammensetzungen der erfindungsgemäßen Verwendung über Flüssigkeiten verabreicht werden sollen, können übliche flüssige Träger verwendet werden. Bevorzugt ist ebenfalls eine Formulierung für Injektionen und Infusionen oder als Suppositorien wie sie auf dem Fachgebiet bekannt und in einschlägigen Standardwerken beschrieben ist.

In der besonders bevorzugten Formulierung als Tabletten werden bevorzugt die Zusatzstoffe Maisstärke, Lactose, Aerosil, Polyvinylpyrrolidon und Magnesiumstearat verwendet. Bei der ebenfalls bevorzugten Ausführungsform als Kapseln werden als Zusatzstoffe bevorzugt Maisstärke, Lactose, Magnesiumstearat und Aerosil verwendet.

Die Zusammensetzungen der erfindungsgemäßen Verwendung können ebenfalls auf an sich bekannte Art und Weise als Depotformulierungen oder zu Arzneimitteln mit verzögerter oder hinhaltender Freisetzung formuliert werden.

Die zu verabreichenden Tagesdosen an Budesonid liegen bei circa 0,5 mg bis 100 mg pro Tag in Abhängigkeit der Schwere der Erkrankung, des Zustandes des Patienten, weiterer vorhandener Erkrankungen, dem Verabreichungsweg und weiterer Parameter, die von dem behandelnden Arzt routinemäßig berücksichtigt werden, bevorzugt liegen die Tagesdosen bei 1 mg bis 50 mg und besonders bevorzugt bei 5 mg bis 20 mg. Die Tagesdosen können auf einmal oder zeitlich versetzt, z.B. dreimal am Tag, verabreicht werden. Entsprechend den vorstehend aufgeführten Tagesdosen enthalten die erfindungsgemäßen Formulierungen bevorzugt 0,5 mg bis 10 mg, besonders bevorzugt 1 mg bis 5 mg Budesonid pro Einheitsdosisform.

Die folgenden Beispiele erläutern die Erfindung

### Formulierungsbeispiel 1: Tabletten zu 5 mg

| | |
|---|---|
| Budesonid | 50 g |
| Maisstärke | 450 g |
| Lactose | 450 g |
| Aerosil | 50 g |

werden gemischt und mit

| | |
|---|---|
| Polyvinylpyrrolidon | 100 g |

gelöst in 500 ml Ethanol (70%ig) befeuchtet.

Die feuchte Masse wird durch ein 1 mm-Sieb geschlagen und getrocknet. Nach erneutem Sieben der getrockneten Masse werden

| | |
|---|---|
| Magnesiumstearat | 30 g |

zugemischt.

Die Mischung wird zu Tabletten von 120 mg verpreßt.

### Formulierungsbeispiel 2: Kapseln zu 3 mg

| | |
|---|---|
| Budesonid | 30 g |
| Maisstärke | 300 g |
| Lactose | 200 g |
| Magnesiumstearat | 30 g |
| Aerosil | 20 g |

werden gemischt, und in Hartgelatinekapseln abgefüllt. Das Füllgewicht beträgt 58 mg.

### Formulierungsbeispiel 3: Injektion 3 mg/ml

| | |
|---|---|
| Budesonid | 3 g |

werden gelöst in

| | |
|---|---|
| Lecithin (USP 23) | 60 g |

Die Lösung wird unter starkem Scheren mit einem Ultraturrax in 1000 ml Wasser (pH 3,5, Citratpuffer, 50 mM) eingetropft. Die entstandene Lösung wird in 1 ml Ampullen abgefüllt und bei 121°C 20 min lang sterilisiert.

### Formulierungsbeispiel 4: Suppositorien 10 mg

| | |
|---|---|
| Budesonid (mikronisiert) | 10 g |

wird in

| | |
|---|---|
| Hartfett | 2000 g |

das auf ca. 45°C aufgeschmolzen ist, suspendiert und in Suppositorienformen zu 2 g ausgegossen. Nach dem Erkalten werden die Suppositorien entnommen.

### Experimentelles Beispiel

Eine Patientin mit der Diagnose PBC wurde über einen längeren Zeitraum mit 3 * 3 mg Budesonid pro Tag behandelt. Vor Therapiebeginn wurden die klinischen Parameter bestimmt, die im Laufe der Therapie weiter verfolgt wurden (Tab. 1). Als klinische Parameter wurden die Enzymaktivitäten der GPT (Alanin-Aminotransferase), der AP (Alkalische Phosphatase), und der LAP (Leucinaminopeptidase) herangezogen. Die GPT ist ein Enzym, das die höchste Aktivität in der Leber besitzt. Ein Anstieg der Serumaktivität dieses Enzyms (normal: bis 22 U/l) weist mit hoher Spezifität auf eine Leberschädigung hin. Eine erhöhte Serumaktivität der AP (normal: bis 170 U/l) tritt bei allen Erkrankungen der Leber und Gallenwege auf, die mit einer Cholestase einhergehen. Ein weiterer Parameter der auf eine cholestatische Lebererkrankung hinweist ist die Serumaktivität der LAP. Ein erhöhter Wert der Enzymaktivität im Serum (normal: 11 - 35 U/l) weist auf eine hepatobiliäre Erkrankung mit obstruktiver und nichtobstruktiver Cholestase hin.

Wie aus Tabelle 1 ersichtlich, sind alle oben beschriebenen Serumaktivitäten bei der Patientin mit PBC vor Behandlung mit Budesonid oberhalb der Normalwerte gewesen.

Bereits nach einmonatiger Behandlung mit täglich 3 * 3 mg Budesonid besserten sich alle Werte bereits deutlich und stiegen auch nach einer 12 monatigen Behandlung nicht wieder an. Während der ganzen Behandlungsdauer traten zudem keinerlei Nebenwirkungen auf.

**Tabelle 1**

| **Serumaktivität verschiedener Leberenzyme [U/l]** | | | |
|---|---|---|---|
| Behandlungszeitraum | GPT [U/l] | AP [U/l] | LAP [U/l] |
| vor Behandlung | 32 | 187 | 53 |
| nach 1 Mon. Therapie | 9 | - | - |
| nach 2 Mon. Therapie | 10 | - | 29 |
| nach 12 Mon. Therapie | 7 | 117 | 29 |

Dies zeigt, daß durch die Behandlung mit Budesonid ein eindeutiger Behandlungserfolg bei cholestatischen Lebererkrankungen erzielt werden kann.

## Patentansprüche

1. Verwendung von Budesonid zur Herstellung eines Arzneimittels zur Behandlung cholestatischer Lebererkrankungen, **dadurch gekennzeichnet, daß** Budesonid nicht zur Unterstützung der Behandlung einer cholestatischen Lebererkrankung mit Ursodesoxycholsäure verabreicht wird.

2. Verwendung nach Anspruch 1, wobei die cholestatische Lebererkrankung primäre biliäre Cirrhose, primäre sclerosierende Cholangitis oder autoimmune Cholangitis ist.

3. Verwendung nach Anspruch 1 oder 2, wobei die Tagesdosis von Budesonid 1 bis 20 mg beträgt.

## Claims

1. Use of budesonide for the production of a pharmaceutical composition for the treatment of cholestatic liver diseases, **characterized in that** budesonide is not administered to support the treatment of a cholestatic liver disease with ursodeoxycholic acid.

2. Use according to claim 1, wherein the cholestatic liver disease is primary biliary cirrhosis, primary sclerosing cholangitis or autoimmune cholangitis.

3. Use according to claim 1 or 2, wherein the daily dose of budesonide is 1 to 20 mg.

## Revendications

1. Utilisation de budésonide pour la fabrication d'un médicament destiné au traitement d'affections hépatiques cholé-statiques, **caractérisée en ce que** le budésonide n'est pas administré pour aider au traitement d'une affection hépatique choléstatique par de l'acide ursodésoxycholique.

2. Utilisation suivant la revendication 1, dans laquelle l'affection hépatique choléstatique est une cirrhose biliaire primaire, une cholangite sclérosante primaire ou une cholangite auto-immune.

3. Utilisation suivant l'une des revendications 1 et 2, dans laquelle la dose journalière de budésonide est de 1 à 20 mg.
